(19) Europäisches Patentamt
European Patent Office
Office européen des brevets

(11) EP 2 716 278 A1

(12) **EUROPEAN PATENT APPLICATION**
published in accordance with Art. 153(4) EPC

(43) Date of publication:
**09.04.2014 Bulletin 2014/15**

(21) Application number: **12792078.3**

(22) Date of filing: **16.05.2012**

(51) Int Cl.:
***A61K 8/55*** *(2006.01)* ***A61K 8/31*** *(2006.01)*
***A61K 8/37*** *(2006.01)* ***A61Q 19/00*** *(2006.01)*

(86) International application number:
**PCT/JP2012/062488**

(87) International publication number:
**WO 2012/165145 (06.12.2012 Gazette 2012/49)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.06.2011 JP 2011122998**

(71) Applicants:
- **Daicel Corporation**
  **Osaka-shi, Osaka 530-0001 (JP)**
- **Nihon University**
  **Tokyo 102-8275 (JP)**

(72) Inventors:
- **SAKANISHI, Yuichi**
  **Ohtake-shi**
  **Hiroshima 739-0695 (JP)**
- **HASHIZAKI, Kaname**
  **Tokyo 102-8275 (JP)**
- **SAITO, Yoshihiro**
  **Tokyo 102-8275 (JP)**
- **TAGUCHI, Hiroyuki**
  **Tokyo 102-8275 (JP)**

(74) Representative: **Grünecker, Kinkeldey, Stockmair & Schwanhäusser**
**Leopoldstrasse 4**
**80802 München (DE)**

(54) **OILY GEL COMPOSITION**

(57) Provided is a gel-forming agent that is easy to prepare and has all the properties including high safety for the living body and the environment, satisfactory gel-forming capability, comfortable feeling upon use, and good handleability. The gel-forming agent includes a lecithin, and a polyglycerol fatty acid ester in an amount of from 30 to 150 parts by weight per 100 parts by weight of the lecithin. The polyglycerol fatty acid ester has a fatty acid residue having 14 or less carbon atoms, has an HLB of 15 or more as determined based on an organic conceptual diagram, and has a degree of glycerol polymerization of from 8 to 40.

EP 2 716 278 A1

## Description

### Technical Field

The present invention relates to a gel-forming agent; and an oil-based gel-like composition containing the gel-forming agent and an oil-phase component.

### Background Art

Gel-forming agents solidify oil-phase components such as animal and vegetable oils, mineral oils, hydrocarbons, and fatty acid esters, through viscosity increasing or gel formation (thickening or gelation). The gel-forming agents are widely used in various areas such as cosmetics, pharmaceuticals, foodstuffs, coating materials, inks, and lubricating oils. Such gel-forming agents should generally have the ability of causing gelation of a target oil-phase component by the addition thereof in a small amount and the ability of giving a gel that is stable over a long period of time. In addition, gel-forming agents for use in some applications should have high safety for the human body and the environment and the ability of forming a gel that has thixotropy and has a good touch.

Customarily known gel-forming agents are exemplified by low-molecular-weight gel-forming agents such as 1,2,3,4-dibenzylidene-D-sorbitol, 12-hydroxystearic acid, and amino acid derivatives; and high-molecular-weight (polymeric) gel-forming agents such as poly(acrylic acid) derivatives and dextrin derivatives. The low-molecular-weight gel-forming agents self-assemble in an oil-phase component to form a huge network structure and immobilize the oil-phase component to form a gel. In contrast, the high-molecular-weight gel-forming agents are intricately entangled to form a network structure to thereby cause the gelation of the oil-phase component.

Independently, the gelation of an oil-phase component by the action of reverse worm-like micelles is also reported (Non Patent Literature (NPL) 1 to 6), although only few reports are available. The reverse worm-like micelle is a type of a self assembly formed by a surfactant and is known to form a network structure in an oil-phase component and to cause gelation of the oil-phase component. The reverse worm-like micelle, as having a hydrophilic environment inside thereof, can contain, for example, a water-soluble drug or enzyme therein and has a feature not available in the above-mentioned gel-forming agents.

As a representative system to form the reverse worm-like micelle, there is reported a three-component mixture system including a lecithin, water, and an oil-phase component of a various kind (NPL 1). In addition, ethylene glycol, formamide, glycerin, bile salts (NPL 3), urea (NPL 4), sucrose fatty acid esters (NPL 5), and D-ribose and D-deoxyribose (NPL 6) are reported as alternative materials for water. Generally, a lecithin forms reverse spherical micelles or reverse elliptical micelles in the oil-phase component. However, typically when a small amount of water is added to the lecithin, the water is bonded to a phosphate group of the lecithin through hydrogen bonding to reduce the interface curvature of the molecular assembly. Probably because of this, the growth of the reverse worm-like micelles occurs.

The gelation of an oil-phase component by the action of an emulsion is also reported in Patent Literature (PTL) 1 as a technique for causing gelation of the oil-phase component, in addition to the techniques as described above. Specifically, the technique gives a gel-like emulsion by preparing one or more surfactants such as lecithins and sucrose fatty acid esters, and adding a higher alcohol, glycerol, and an oil-phase component to the surfactants.

### Citation List

#### Patent Literature

PTL 1: Japanese Unexamined Patent Application Publication (JP-A) No. H05-4911

#### Non Patent Literature

NPL 1: P. L. Luisi et al., Colloid & Polymer Science, vol. 268, p. 356 (1990)
NPL 2: Yu. A. Shchipunov, Colloids and Surfaces A, vol. 183-185, p. 541 (2001)
NPL 3: S. H. Tung et al. Journal of the American Chemical Society, vol. 128, p. 5751 (2006)
NPL 4: K. Hashizaki et al., Colloid & Polymer Science, vol. 287, p. 927 (2009)
NPL 5: K. Hashizaki et al., Colloid & Polymer Science, vol. 287, p. 1099 (2009)
NPL 6: K. Hashizaki et al., Chemistry Letters, vol. 38, p. 1036 (2009)

## Summary of Invention

### Technical Problem

Of the low-molecular-weight gel-forming agents, 1,2,3,4-dibenzylidene-D-sorbitol is an excellent compound that can cause gelation of oil-phase components of various types. This compound, however, has not yet been practically used because it forms benzaldehyde upon decomposition and has poor safety. The 12-hydroxystearic acid has poor thixotropy, although commercially available as a gel-forming agent for waste deep-frying oil. The amino acid derivatives as gel-forming agents are hardly soluble in the oil-phase component and require complicated operations such as high-temperature heating and/or long-term stirring to be dissolved therein. In addition, such operations may disadvantageously cause quality change of other components to be incorporated into the gel. The dextrin derivatives as high-molecular-weight gel-forming agents need to be added in a high concentration for the gelation, cause "sticky feeling" peculiar to polymers, and give a poor feeling upon use. The poly(acrylic acid) derivatives provide good thickening and gel formation even in a small amount, but when used for the skin, they cause a "sticky feeling" peculiar to polymers and give a poor feeling upon use.

Next, disadvantages of customary reverse worm-like micelles will be illustrated. Assume that the reverse worm-like micelle including a lecithin, water, and an oil-phase component is used as an exemplary representative reverse worm-like micelle. This reverse worm-like micelle, as containing water as a component, cannot incorporate a drug or another substance which is susceptible to hydrolysis. A customary gel-forming agent, when employing glycerol being liquid at room temperature as an alternative material for water, exhibits insufficient gel-forming capability. The gel-forming agent, when employing, instead of water, ethylene glycol or formamide being liquid at room temperature as with glycerol, is disadvantageously hardly applied to the human body because of being highly irritative typically to the skin, eyes, and mucous membranes. The gel-forming agent, when employing a substance that is solid at room temperature, such as a bile salt, urea, a sucrose fatty acid ester, or D-ribose, disadvantageously requires a preparation technique that is more complicated than that required by a gel-forming agent using a liquid component. Accordingly, there has not yet been obtained a gel-forming agent which is easy to prepare and has all of properties such as high safety for the living body and the environment, satisfactory gel-forming capability, comfortable feeling upon use, and good handleability.

PTL 1 discloses the gel-like emulsion prepared by blending one or more surfactants such as lecithins and sucrose fatty acid esters with a higher alcohol, glycerol, and an oil-phase component. This gel has lower elasticity than those of the gels formed by the gel-forming agents or the reverse worm-like micelles, thereby disadvantageously has poor handleability due to easiness to drip, and fails to exhibit satisfactory effects if either one of the higher alcohol and glycerol is not incorporated.

Accordingly, an object of the present invention is to provide a gel-forming agent which is easy to prepare and has all of the properties including high safety for the living body and the environment, satisfactory gel-forming capability, comfortable feeling upon use, and good handleability.

Another object of the present invention is to provide an oil-based gel-like composition which includes the gel-forming agent having the superior properties and an oil-phase component and is highly stable as a gel.

### Solution to Problem

Gel-forming agents and thickened gel-like compositions are used in various areas such as cosmetics, pharmaceuticals, foodstuffs, coating materials, inks, and lubricating oils. In this case, they should have, for example, the ability of causing gelation of a target oil-phase component by adding a small amount thereof and the ability of providing a gel which is stable over a long period of time, as mentioned above. In some applications, the gel-forming agents and the thickened gel-like compositions should also have extremely high safety for the human body and the ability of forming a gel that has thixotropy and has a good touch. However, the customary techniques failed to give a satisfactory gel-forming agent having all of the required properties.

Under such circumstances, the present inventors have made intensive investigations to achieve the objects and have employed a three-component mixture system including a lecithin, a polyglycerol fatty acid ester, and an oil-phase component and have successfully obtained a gel-forming agent and a thickened gel-like composition each including reverse worm-like micelles.

The lecithin for use herein is an amphoteric phospholipid having two alkyl chains and is used as a foodstuff emulsifier for emulsifying dairy products and reducing the viscosity of chocolate; and also widely used in other applications such as pharmaceutical preparations. The lecithin has high safety for the living body and the environment. Independently, the polyglycerol fatty acid ester has strong hydrogen-bonding capability and high safety and is thereby used as a cosmetic humectant and a foodstuff emulsifier. The present inventors have employed a three-component mixture system including a lecithin, a polyglycerol fatty acid ester, and an oil-phase component for the preparation of a gel-forming agent capable of forming reverse worm-like micelles. As a result, they have found that the blending ratio among the three components

suitable for gel formation varies depending on the fatty acid residue, the HLB calculated based on the organic conceptual diagram, and the degree of polymerization each of the polyglycerol fatty acid ester.

Specifically, the present invention provides:

an oil-based gel-like composition including 1 to 30 percent by weight of a gel-forming agent; and 70 to 99 percent by weight of an oil-phase component,

in which:

the gel-forming agent includes a lecithin, and a polyglycerol fatty acid ester in an amount of from 30 to 150 parts by weight per 100 parts by weight of the lecithin; and
the polyglycerol fatty acid ester has a fatty acid residue having 14 or less carbon atoms, has an HLB of 15 or more as calculated based on the organic conceptual diagram, and has a degree of glycerol polymerization of from 8 to 40.

In the oil-based gel-like composition,
the oil-based gel-like composition preferably has a zero-shear viscosity of 50 Pa•s or more, where the zero-shear viscosity is determined from a viscosity curve plotted through rheological measurement.

The present invention also provides:

a gel-forming agent including a lecithin, and a polyglycerol fatty acid ester in an amount of from 30 to 150 parts by weight per 100 parts by weight of the lecithin,
in which the polyglycerol fatty acid ester has a fatty acid residue having 14 or less carbon atoms, has an HLB of 15 or more as calculated based on the organic conceptual diagram, and has a degree of glycerol polymerization of from 8 to 40.

As used herein the term "oil-based gel-like composition" refers to a gel-like composition including no water, or including water only in a trace amount (e.g., 1 percent by weight or less, and preferably 0.2 percent by weight or less).

## Advantageous Effects of Invention

The gel-forming agent according to the present invention, as having the configuration as described above, is easy to prepare, is highly safe for the living body and the environment, and has all of properties, i.e., satisfactory gel-forming capability, comfortable feeling upon use, and good handleability. The gel-forming agent also has satisfactory transparency.

The oil.-based gel-like composition according to the present invention, as having the configuration as described above, is highly safe for the living body and the environment, gives comfortable feeling upon use, is highly stable as a gel, and has satisfactory transparency.

## Description of Embodiments

### Lecithin

The lecithin is a lipid product that includes a phosphatidylcholine as a principal component, is widely distributed in living bodies such as natural animals, plants (vegetables), and microorganisms, and is known to be contained in a large amount typically in the liver, yolks, soybeans, and yeasts. Representative lecithins include yolk lecithin and soybean lecithin. Each of different lecithins can be used alone or in combination. The lecithin for use herein is preferably one having a phosphatidylcholine content of from about 55 to about 99 percent by weight. The lecithin having a phosphatidylcholine content within this range is easily formed into cream, has suitable consistency, remains on the skin without falling off when applied to the skin, and gives comfortable feeling upon use. Natural lecithins are present only in the L-$\alpha$-form, but lecithins of other forms are also usable. Such a natural lecithin is susceptible to oxidation and unstable. To mitigate this disadvantage, the natural lecithin is preferably hydrogenated according to a known procedure before use. The term "lecithin"' in the present invention also includes such a hydrogenated lecithin.

The term "phosphatidylcholine" refers to an ester obtained by allowing glycerol (glycerin) to react with an unsaturated fatty acid and a phosphoric acid. Protons of the phosphoric acid are substituted by choline serving as an amine functional group. The "phosphatidylcholine" herein also includes a phosphatidylcholine with unsaturated bond(s) being hydrogenated.

The phosphatidylcholine for use in the present invention is especially defined by General Formula (I) mentioned below. In the formula, $R_1$ and $R_2$ independently represent aliphatic hydrocarbon groups derived from (corresponding to)

saturated or unsaturated fatty acids each having 4 to 24 carbon atoms. Specifically, $R_1$ and $R_2$ independently represent saturated or unsaturated aliphatic hydrocarbon groups each having 3 to 23 carbon atoms. These groups may each be a straight or branched chain group and may be substituted with one or more of hydroxyl functional groups and/or amine functional groups. X represents a choline residue. Each of different compounds represented by Formula (I) may be used alone or in combination as the phosphatidylcholine.

[Chem. 1]

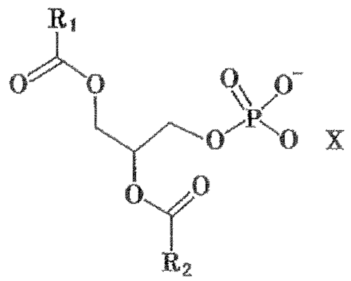

(I)

In one embodiment of the present invention, fatty acids ($R_1COOH$ and $R_2COOH$) corresponding to $R_1$ and $R_2$ are selected typically from butyric acid, caproic acid, caprylic acid, capric acid, caproleic acid, lauric acid, lauroleic acid, myristic acid, tyristoleic acid, palmitic acid, palmitoleic acid, stearic acid, oleic acid, linoleic acid, linolenic acid, arachidic acid, isostearic acid, dihydroxystearic acid, and ricinoleic acid.

A non-hydrogenated phosphatidylcholine (PC) suitable to prepare the composition according to the present invention can be derived from a "natural" substance or a "synthetic" substance.

The "natural" phosphatidylcholine (PC) can be obtained by extraction from an animal source or plant source such as soybeans, sunflowers, or eggs. A non-hydrogenated phosphatidylcholine derived from a natural substance such as soybeans generally includes palmitic acid, stearic acid, palmitoleic acid, oleic acid, linoleic acid, linolenic acid, and fatty acids probably having 20 to 22 carbon atoms, as fatty acids serving to esterify the glycerol.

Polyglycerol Fatty Acid Ester

The polyglycerol fatty acid ester for use herein is produced by any of various methods. Exemplary methods include (1) a method of subjecting glycerol to addition polymerization with glycidol, and esterifying the resulting compound with a fatty acid; (2) a method of adding glycidol to a fatty acid; as a method relating to the method (2), (3) a method of adding glycidol with hydroxyl group being protected to glycerol, deprotecting the hydroxyl group, repeating these processes until the resulting compound has an arbitrary degree of polymerization, and esterifying this with a fatty acid; and (4) a method of thermally condensing glycerol in the presence of a base, and esterifying the resulting compound with a fatty acid. Among them, the method (1) is most preferred as a method to produce a polyglycerol fatty acid ester, and the resulting polyglycerol fatty acid ester is advantageously usable in the gel-forming agent of the oil-based gel-like composition.

The polyglycerol fatty acid ester for use herein has a fatty acid residue having 14 or less carbon atoms. The fatty acid residue preferably has 6 to 14 carbon atoms. A fatty acid residue having more than 14 carbon atoms may cause the composition to exist not as a gel composition but as a transparent solution. The fatty acid residue may have a straight or branched chain structure, but preferably has a straight chain structure. The fatty acid residue may be a residue of a saturated fatty acid or unsaturated fatty acid, but is preferably a residue of a saturated fatty acid. Examples of the fatty acid residue include residues of hexanoic acid, heptanoic acid, octanoic acid, nonanoic acid, decanoic acid, undecanoic acid, dodecanoic acid, and tetradecanoic acid. The polyglycerol fatty acid ester may have each of different fatty acid residues alone or in combination.

The polyglycerol fatty acid ester for use herein has a degree of glycerol polymerization of from 8 to 40 and particularly preferably has a degree of polymerization of from 8 to 20. A polyglycerol fatty acid ester having a degree of glycerol polymerization of less than 8 may fail to contribute to the formation of a stable gel. In contrast, a polyglycerol fatty acid ester having a degree of glycerol polymerization of more than 40 may cause the composition to be not a transparent gel, but an emulsified composition, and this may impede the production of gel itself.

Such polyglycerol fatty acid esters as mentioned above each have an HLB of 15 or more, more preferably has an HLB of from 15 to 20, as calculated based on the organic conceptual diagram. A polyglycerol fatty acid ester having an HLB of less than 15 as calculated based on the organic conceptual diagram may cause the composition to exist not a gel composition, but a transparent solution. A polyglycerol fatty acid ester having an HLB of from 15 to 20 as calculated based on the organic conceptual diagram can prevent the formation of an emulsified composition but facilitates the

formation of a gel composition. The "HLB" as calculated based on the organic conceptual diagram is herein defined as a value determined according to Formula (A) as follows:

$$HLB=\Sigma(\text{Inorganic values})/\Sigma(\text{Organic values})\times 10 \quad (A)$$

(Reference: "Formulation Design with Organic Conceptual Diagram", Nihon Emulsion Co., Ltd.)

Each of polyglycerol fatty acid esters having different degrees of polymerization can be used alone or in combination.

Gel-forming Agent

The gel-forming agent according to the present invention includes the lecithin; and the polyglycerol fatty acid ester in an amount of from 30 to 150 parts by weight per 100 parts by weight of the lecithin.

The lecithin is preferably one containing a phosphatidylcholine in a content of from 55 to 99 percent by weight, as described above. The gel-forming agent therefore preferably includes the polyglycerol fatty acid ester in an amount of from 30 to 150 parts by weight per 55 to 99 parts by weight (e.g., 75 parts by weight) of the phosphatidylcholine.

The oil-based gel-like composition can contain the gel-forming agent in a content of from 1 to 30 percent by weight, preferably from 5 to 20 percent by weight, and particularly preferably from 10 to 15 percent by weight, based on the total amount of the oil-based gel-like composition. The gel-forming agent, if contained in the oil-based gel-like composition in an excessively small content (total content of the lecithin and the polyglycerol fatty acid ester) based on the total amount of the composition, may cause insufficient gel formation and fail to give a stable oil-based gel-like composition. In contrast, the gel-forming agent, if contained in the oil-based gel-like composition in an excessively high content based on the total amount of the composition, may provide no merit of use in such a large amount and be uneconomical, because the gel-forming ability and the moisturizing/water-holding action reach a peak. To prevent these, the oil-based gel-like composition preferably contains the gel-forming agent in a content within the above-specified range.

The lecithin content based on the total amount of the oil-based gel-like composition can be calculated from the above description and is preferably in the range of from 1 to 20 percent by weight, and particularly preferably in the range of from 6 to 15 percent by weight. The lecithin, if contained in an excessively low content, may often cause insufficient gel formation and fail to give a stable oil-based gel-like composition. In contrast, the lecithin, if contained in an excessively high content, may provide no merit of use in such a large amount and be uneconomical, because the gel-forming ability and the moisturizing/water-holding action reach a peak. To prevent these, the oil-based gel-like composition preferably contains the lecithin within the above-specified range. The oil-based gel-like composition may contain the phosphatidylcholine in a content of typically from 0.75 to 19 percent by weight, and preferably from 4 to 14 percent by weight, based on the total amount of the composition.

The polyglycerol fatty acid ester content based on the total amount of the oil-based gel-like composition can also be calculated from the above description, and is preferably in the range of from 0.5 to 30 percent by weight and particularly preferably in the range of from 1 to 20 percent by weight. The polyglycerol fatty acid ester, if contained in an excessively low content, may fail to give a stable oil-based gel-like composition; and, if contained in an excessively high content, may provide no merit of use in such a large amount and be uneconomical, because the gel-forming ability and the moisturizing/water-holding action reach a peak. To prevent these, the oil-based gel-like composition preferably contains the polyglycerol fatty acid ester in a content within the above-specified range.

Oil Phase Component

The oil-phase component for use herein includes, as a principal component, a polar oil alone, or a mixture of a polar oil with a nonpolar oil, or a nonpolar oil alone. The nonpolar oil is exemplified by squalane, petrolatum, liquid paraffin, and other hydrocarbons; and chain or cyclic silicone oils. The polar oil is exemplified by olive oil and other oils and fats; lanolin and other waxes; isopropyl myristate, decyl oleate, glyceryl tri-(2-ethylhexanoate), and other esters [e.g., esters between a fatty acid having 8 or more carbon atoms (preferably one having 8 to 25 carbon atoms) with an alcohol]; oleic acid, lauric acid, and other higher fatty acids [e.g., fatty acids each having 12 or more carbon atoms, of which fatty acids each having 12 to 25 carbon atoms are preferred]; and cetanol and other higher alcohols which are solid at room temperature [e.g., alcohols each having 12 or more carbon atoms, of which alcohols having 12 to 25 carbon atoms are preferred]. Each of different oil-phase components may be used alone or in combination in a total content in the range of from 70 to 98 percent by weight based on the total amount of the oil-based gel-like composition. An oil-based gel-like composition containing the oil-phase component content(s) in a low content of less then 70 percent by weight may contain the gel-forming agent in an excessively high content; and an oil-based gel-like composition containing

the oil-phase component in a content of more than 98 percent by weight may suffer from poor stability as a gel and be uneconomical. To prevent these, the oil-based gel-like composition preferably contains the oil-phase component(s) in a content within the above-specified range. The oil-based gel-like composition contains the oil-phase component(s) in a content of preferably from 80 to 95 percent by weight, and more preferably from 85 to 90 percent by weight, based on the total amount of the composition.

Other Components

The lecithin by itself is effective as cosmetics which spread into every corner of the keratinized skin layer (keratinized layer) and soften the aged and keratinized skin layer. The oil-based gel-like composition, when used as skin cosmetics, may further contain any of other components such as vitamin B, vitamin E, and various flavors for better effects as the skin cosmetics. Among such additional components, ascorbic acid is particularly effective. The ascorbic acid, as having a pH of about 2, helps the cosmetics to have a lower pH and exhibits a keratolytic action to remove the old keratinized layer. An ascorbic acid derivative such as ascorbyl palmitate is usable for more stable incorporation of ascorbic acid. The oil-based gel-like composition may further contain an antimicrobial component such as hinokithiol, fucoidan, or salicylic acid. The composition in this case can react with a fungus or bacterium present in the keratinized layer. In addition or alternatively, the oil-based gel-like composition may further contain a vegetable anti-inflammatory/moisturizing component such as glucyrrhizic acid. The composition in this case is expected to exhibit a sedative/moisturizing action in the keratinized layer which becomes sclerotic and undergoes laceration and inflammation. For the prevention of sticky feeling, the oil-based gel-like composition may further contain any of powders such as silica powders, silicon powders, and alkyl acrylate copolymer powders.

The oil-based gel-like composition according to the present invention may further contain, in addition to the components as mentioned above, one or more components for regular cosmetics uses. The components are exemplified by flavors, coloring agents or pigments, antiseptic agents, antioxidants, anti-inflammatory agents, ultraviolet absorbers, ultraviolet reflectors, and pH adjusters; as well as medicinal components such as hyaluronic acid, allantoin, vitamins, amino acids, and placental extract according to necessity. Each of such components may be suitably incorporated alone or in combination.

The oil-based gel-like composition according to the present invention may contain such other components than the gel-forming agent and the oil-phase component in a content of generally 29 percent by weight or less (e.g., from 0.1 to 29 percent by weight), preferably 20 percent by weight or less (e.g., from 0.1 to 20 percent by weight), and more preferably 10 percent by weight or less (e.g., from 0.1 to 10 percent by weight) based on the total amount of the composition.

The oil-based gel-like composition obtained according to the present invention generally remains stable over a long period of time typically of three months or longer. The composition, as being verified to have appropriate elasticity through rheological measurement, is assessed to be resistant to dripping and to have good handleability. In addition, the composition has thixotropy and satisfactorily spreads when applied typically to the skin.

A zero-shear viscosity of the oil-based gel-like composition according to the present invention is herein determined based on a viscosity curve plotted through rheological measurement. The zero-shear viscosity is defined as follows. Specifically, of regions where the shear rate becomes closer and closer to zero, there is a region where even a non-Newtonian fluid can be regarded as an analogue to a Newtonian fluid. In this region, the viscosity of the fluid does not vary and indicates a constant value. The viscosity $\eta$ herein is defined as the zero-shear viscosity determined based on a viscosity curve plotted through rheological measurement. Though not critical, the oil-based gel-like composition has the zero-shear viscosity of preferably 50 Pa·s or more, and particularly preferably 100 Pa·s or more. This range is preferred from the viewpoints typically of gel stability, gel touch, feeling upon use, and handleability. An upper limit of the zero-shear viscosity is not critical, may vary depending on the intended use, but is typically 2000 Pa·s, and preferably 1000 Pa·s.

EXAMPLES

The present invention will be illustrated in further detail with reference to several examples below, which are by no means intended to limit the scope of the invention.

EXAMPLES 1 TO 5 AND COMPARATIVE EXAMPLES 1 TO 14

Oil-based gel-like compositions were prepared by blending a lecithin, a polyglycerol fatty acid ester, and n-decane (as an oil-phase component) in ratios given in Tables 1 to 3. The resulting oil-based gel-like compositions were each subjected to a rheological measurement so as to evaluate viscosity increase and gel formation. Independently, the transparency of each composition was determined. The results are indicated in Tables 1 to 3. Figures in the tables

represent blending ratios (percent by weight) of respective components. Reagents, preparation methods, and evaluation methods used herein are as follows.

Reagents

Soybean lecithin was used as the lecithin. The soybean lecithin was a product supplied by Avanti Polar Lipids, Inc and having a phosphatidylcholine concentration of 95%. An analytical grade reagent supplied by Kanto Chemical Co., Inc. was used as intact as the n-decane. Polyglycerol fatty acid ester products supplied by Daicel Chemical Industries Ltd. were used as intact as the polyglycerol fatty acid esters.

Preparation Method

Necessary amounts of the lecithin, polyglycerol fatty acid ester, and n-decane were encapsulated in a bottle and stirred overnight using a magnetic stirrer. The resulting mixture was left stand in a thermostat at 25°C for several days to reach equilibrium and yielded each sample.

Evaluations

The compositions obtained according to Examples and Comparative Examples were respectively evaluated by methods as follows.

(1) Rheological Measurement

The rheological measurement was performed using a viscosity and viscoelasticity measuring instrument (rheometer) (HAAKE RheoStress 600) equipped with a cone-and-plate sensor (sensor used had a diameter of 60 mm with a cone angle of 1°, or a diameter of 35 mm with a cone angle of 1°, 2°, or 4°) and a Peltier temperature controller. All the measurements were performed in a steady flow viscosity measurement mode at 25°C, in which the viscosity was measured at different shear rates varying in a log scale from 0.001 to 100 ($s^{-1}$), based on which viscosity curves were plotted. Each plot employed a value obtained when the torque value variation of the instrument was settled within the range of 5% and the data became stable.

(2) Viscosity Increase and Gel Formation Evaluation

The polyglycerol fatty acid ester, lecithin, and n-decane were blended with each other in ratios given in Tables 1 to 3 to prepare oil-based gel-like compositions. The resulting oil-based gel-like compositions were each subjected to a rheological measurement. The zero-shear viscosity $\eta 0$ of each composition was determined based on a viscosity curve plotted through the rheological measurement. As is described above, of regions where the shear rate becomes closer and closer to zero, there is a region where even a non-Newtonian fluid can be regarded as an analogue to a Newtonian fluid. A constant viscosity $\eta$ in this region was defined as a zero-shear viscosity $\eta 0$. The viscosity herein became constant at a shear rate of 0.1 ($s^{-1}$) or less, and this constant viscosity was defined as the zero-shear viscosity $\eta 0$.

The viscosity increase and gel formation (thickening and gelation) was evaluated as follows based on the zero-shear viscosity $\eta 0$ (Pa•s). The results are indicated in Tables 1 to 3.

Excellent (Exc): Zero-shear viscosity $\eta 0$ of 100 Pa•s or more
Good: Zero-shear viscosity $\eta 0$ of from 50 Pa•s to less than 100 Pa•s
Fair: Zero-shear viscosity $\eta 0$ of from 20 Pa•s to less than 50 Pa•s
Poor: Zero-shear viscosity $\eta 0$ of less than 20 Pa•s

(3) Transparency Evaluation

The transparency was determined as follows by visual observation for the oil-based gel-like compositions obtained in Examples 1 to 5 and Comparative Examples 1 to 14. The results are indicated in Tables 1 to 3.

Excellent (Exc): Transparent

Good: Translucent

Fair: Cloudy

Poor: Separated into two phases

[Table 1]

| | HLB | Examples | | | | |
|---|---|---|---|---|---|---|
| | | 1 | 2 | 3 | 4 | 5 |
| Decaglycerol hexanoic acid ester | 18.61 | 5 | | | | |
| Decaglycerol decanoic acid ester | 1674 | | 7 | 8 | | |
| Decaglycerol tetradecanoic acid ester | 1522 | | | | 14 | 15 |
| Lecithin | | 10 | 10 | 10 | 10 | 10 |
| Decane | | 85 | 83 | 82 | 76 | 75 |
| Transparency | | Exc | Exc | Exc | Exc | Exc |
| Viscosity increase | | Good | Good | Exc | Fair | Fair |

[Table 2]

| | HLB | Comparative Examples | | | | | | |
|---|---|---|---|---|---|---|---|---|
| | | 1 | 2 | 3 | 4 | 5 | 6 | 7 |
| Hexaglycerol hexanoic acid ester | 17.92 | 4 | 5 | | | | | |
| Hexaglycerol decanoic acid ester | 15.38 | | | 8 | 10 | 12 | 14 | 16 |
| Lecithin | | 10 | 10 | 10 | 10 | 10 | 10 | 10 |
| Decane | | 86 | 85 | 82 | 80 | 78 | 76 | 74 |
| Transparency | | Exc | Exc | Exc | Exc | Exc | Exc | Exc |
| Viscosity increase | | Poor | Poor | Poor | Poor | Poor | Poor | Poor |

(Table 3]

| | HLB | Comparative Examples | | | | | | |
|---|---|---|---|---|---|---|---|---|
| | | 8 | 9 | 10 | 11 | 12 | 13 | 14 |
| Decaglycerol octadecanoic acid ester | 13.95 | 6 | 8 | 10 | 12 | 14 | 16 | 18 |
| Lecithin | | 10 | 10 | 10 | 10 | 10 | 10 | 10 |
| Decane | | 84 | 82 | 80 | 78 | 76 | 74 | 72 |
| Transparency | | Exc | Exc | Exc | Exc | Exc | Exc | Exc |
| Viscosity increase | | Poor | Poor | Poor | Poor | Poor | Poor | Poor |

Industrial Applicability

Gel-forming agents and thickened gel-like compositions obtained according to embodiments of the present invention feature reverse worm-like micelles. They are very safe for the human body and the environment and are therefore usable typically as cosmetics, pharmaceuticals, foodstuffs, detergents, deodorants, bath additives, flavoring agents, and deodorizers in various products that are gelatinous at room temperature. They are particularly suitable for use in cosmetics and pharmaceuticals. The cosmetics are exemplified by cream, milky lotions, lotions, cleansing cosmetics, bath cosmetics, moisturizing cosmetics, blood circulation promotion and massaging agents, face pack cosmetics, and hair cosmetics. The pharmaceuticals are exemplified by ointments, molded cataplasms, sustained-release preparation bases, transdermal therapeutic drugs, drug delivery system carriers, and gels for electrophoresis.

## Claims

1. An oil-based gel-like composition comprising 1 to 30 percent by weight of a gel-forming agent; and 70 to 99 percent by weight of an oil-phase component,
wherein:

    the gel-forming agent comprises a lecithin; and a polyglycerol fatty acid ester in an amount of from 30 to 150 parts by weight per 100 parts by weight of the lecithin; and
    the polyglycerol fatty acid ester comprises a fatty acid residue having 14 or less carbon atoms, has an HLB of 15 or more as calculated based on an organic conceptual diagram, and has a degree of glycerol polymerization of from 8 to 40.

2. The oil-based gel-like composition according to claim 1, wherein the oil-based gel-like composition has a zero-shear viscosity of 50 Pa•s or more, the zero-shear viscosity determined from a viscosity curve plotted through rheological measurement.

3. A gel-forming agent comprising: a lecithin; and a polyglycerol fatty acid ester in an amount of from 30 to 150 parts by weight per 100 parts by weight of the lecithin,
wherein the polyglycerol fatty acid ester comprises a fatty acid residue having 14 or less carbon atoms, has an HLB of 15 or more as calculated based on an organic conceptual diagram, and has a degree of glycerol polymerization of from 8 to 40.

**INTERNATIONAL SEARCH REPORT**

International application No.
PCT/JP2012/062488

A. CLASSIFICATION OF SUBJECT MATTER
*A61K8/55*(2006.01)i, *A61K8/31*(2006.01)i, *A61K8/37*(2006.01)i, *A61Q19/00*(2006.01)i

According to International Patent Classification (IPC) or to both national classification and IPC

B. FIELDS SEARCHED

Minimum documentation searched (classification system followed by classification symbols)
A61K8/55, A61K8/31, A61K8/37, A61Q19/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2012 |
| Kokai Jitsuyo Shinan Koho | 1971-2012 | Toroku Jitsuyo Shinan Koho | 1994-2012 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| A | JP 59-76530 A (Mitsuo MATSUMOTO), 01 May 1984 (01.05.1984), entire text (Family: none) | 1-3 |
| A | JP 2003-212750 A (Yugen Kaisha Nonokawa Shoji), 30 July 2003 (30.07.2003), entire text (Family: none) | 1-3 |
| A | JP 2005-320285 A (Kose Corp.), 17 November 2005 (17.11.2005), entire text (Family: none) | 1-3 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 26 July, 2012 (26.07.12) | 07 August, 2012 (07.08.12) |

| Name and mailing address of the ISA/ | Authorized officer |
|---|---|
| Japanese Patent Office | |
| Facsimile No. | Telephone No. |

Form PCT/ISA/210 (second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP H054911 A


### Non-patent literature cited in the description


- **P. L. LUISI et al.** *Colloid & Polymer Science,* 1990, vol. 268, 356
- **YU. A. SHCHIPUNOV.** *Colloids and Surfaces A,* 2001, vol. 183-185, 541
- **S. H. TUNG et al.** *Journal of the American Chemical Society,* 2006, vol. 128, 5751
- **K. HASHIZAKI et al.** *Colloid & Polymer Science,* 2009, vol. 287, 927
- **K. HASHIZAKI et al.** *Colloid & Polymer Science,* 2009, vol. 287, 1099
- **K. HASHIZAKI et al.** *Chemistry Letters,* 2009, vol. 38, 1036